# EUROPÄISCHE PATENTSCHRIFT

(11) **EP 0 653 416 B1**
(45) Veröffentlichungstag und Bekanntmachung des Hinweises auf die Patenterteilung: **24.09.1997**
(21) Anmeldenummer: 94117399.9
(22) Anmeldetag: 04.11.1994
(51) Int. Cl.: C07C 231/02

(54) **Verfahren zur Herstellung von Benzoylaminoanthrachinonverbindungen**
Process for the preparation of benzoyl amino anthraquinone compounds
Procédé pour la préparation de composés de benzoyl-amino-anthraquinone

(30) Priorität: 11.11.1993 DE 4338487
(43) Veröffentlichungstag der Anmeldung: 17.05.1995
(73) Patentinhaber: BASF Aktiengesellschaft, 67063 Ludwigshafen (DE)
(72) Erfinder: Bergmann, Udo, Dr., D-64625 Bensheim (DE); Hoch, Helmut, Dr., D-67273 Weisenheim (DE); Reinhold, Kohlhaupt, Dr., D-67227 Frankenthal (DE)

(56) Entgegenhaltungen:
- EP-A- 0 099 071
- EP-A- 0 326 866
- EP-A- 0 382 053
- DE-A- 3 325 277
- US-A- 3 651 099

## Beschreibung

Die vorliegende Erfindung betrifft ein neues Verfahren zur Herstellung von Benzoylaminoanthrachinonverbindungen (I) durch Umsetzung von mindestens eine primäre Aminogruppe enthaltenden Anthrachinonverbindungen (II) mit Benzoylhalogeniden (III) in Gegenwart eines organischen Lösungsmittels.

Benzoylaminoanthrachinone stellen wichtige Vorprodukte für Küpenfarbstoffe dar oder sind bereits selbst bekannte Küpenfarbstoffe, die vielfach zum Färben oder Bedrucken von Baumwolle oder Baumwollmischgeweben verwendet werden.

Die Herstellung der Benzoylaminoanthrachinone erfolgt bekanntermaßen durch Umsetzung eines durch mindestens eine primäre Aminogruppe substituierten Anthrachinons oder Anthrachinoids mit einem Benzoylhalogenid, wobei dem Reaktionsgemisch in einigen Fällen auch säurebindende Mittel zugesetzt werden.

Allgemein übliche Lösungsmittel für die Umsetzung sind o-Dichlorbenzol (FIAT Final Report 1313 II, Seite 139, BIOS Final Report 1493, Seite 32, US-A-3 651 099), Trichlorbenzol (FIAT Final Report 1313 II, Seite 72 und 73) und Nitrobenzol (FIAT Final Report 1313 II, Seite 26 bis 27 und 72; Ullmanns Encyclopädie der Technischen Chemie, 4. Auflage, Band 7, Seite 595 und 596 (1974); EP-A-99 071; DE-A-33 25 277).

Die bekannten Verfahren haben jedoch den Nachteil, daß sie aufgrund der verwendeten Lösungsmittel besondere, kostspielige Maßnahmen erfordern. Die Halogenbenzole sind biologisch nur schwer abbaubar und müssen daher unter hohem technischen Aufwand aus dem Abwasser, der Abluft und natürlich auch aus den Reaktionsprodukten entfernt werden. Nitrobenzol ist zwar biologisch besser abbaubar, jedoch ist aufgrund seiner gesundheitsschädigenden Wirkung eine aufwendige Absicherung der Reaktionsapparatur und seine restlose Entfernung aus den Produkten, die nur durch zeit- und energieaufwendige Reinigungsprozesse zu erreichen ist, unumgänglich.

Weiterhin werden in den EP-A-326 866 und 382 053 Kondensationsreaktionen von Aminoanthrachinonen mit Anthrachinoncarbonsäurechloriden beschrieben, die in Benzoesäuremethylester durchgeführt werden.

Der Erfindung lag die Aufgabe zugrunde, den genannten Mängeln abzuhelfen und die Herstellung von Benzoylaminoanthrachinonen in vorteilhafter und wirtschaftlicher Weise zu ermöglichen.

Demgemäß wurde ein Verfahren zur Herstellung von Benzoylaminoanthrachinonverbindungen (I) durch Umsetzung von mindestens eine primäre Aminogruppe enthaltenden Anthrachinonverbindungen (II) mit Benzoylhalogeniden (III) in Gegenwart eines organischen Lösungsmittels gefunden, welches dadurch gekennzeichnet ist, daß man als organisches Lösungsmittel einen Benzoesäure-C₁-C₆-alkylester verwendet.

Beim erfindungsgemäßen Verfahren werden als Lösungsmittel die unter den Reaktionsbedingungen flüssigen Benzoesäure-C₁-C₆-alkylester, z.B. Benzoesäurehexyl-, -n-, -iso- und -sek.-pentyl-, -n-, -iso- und -sek.-butyl- und -n- und -isopropylester, bevorzugt Benzoesäureethylester und besonders bevorzugt Benzoesäuremethylester, eingesetzt. Selbstverständlich können auch Mischungen dieser Ester als Lösungsmittel eingesetzt werden.

Die Menge an Lösungsmittel beträgt in der Regel 1 bis 25, vorzugsweise 3 bis 15 kg pro kg Benzoylaminoanthrachinon (I).

Die beim erfindungsgemäßen Verfahren als Ausgangsverbindungen (II) dienenden Aminoanthrachinone können eine oder mehrere Aminogruppen und auch weitere Substituenten tragen sowie an ein Ringsystem, das heterocyclisch sein kann, anelliert sein.

Geeignete Substituenten sind z.B. Hydroxy, Halogen, insbesondere Chlor und Brom, C₁-C₄-Alkyl, vor allem Methyl und Ethyl, C₁-C₄-Alkoxy, vor allem Methoxy und Ethoxy, und aromatische Reste wie Phthaloylbenzthiazol und Anthrachinonylamino. Im letztgenannten Fall liegt (II) als Anthrimid vor.

Beispiele für bevorzugte Ausgangsverbindungen (II) sind neben 2-Aminoanthrachinon, 2-Amino-3-bromanthrachinon, 2-Amino-3-methylanthrachinon, 2-Amino-3-hydroxyanthrachinon und 1-Amino-4-hydroxyanthrachinon vor allem 1-Aminoanthrachinon und 1,4- und 1,5-Diaminoanthrachinon sowie 1,4-Diamino-2,2'-(5',6'-phthaloylbenzthiazolyl)-anthrachinon und 4,4'-Diamino-1,1'-dianthrimid und (9)-Benzo-5-aminoanthrachinoyl (2,1-h)-8-azapyrenon-3.

Die als Ausgangsverbindungen (III) verwendeten Benzoylhalogenide, insbesondere -chloride, auch -bromide und -fluoride, können ebenfalls Substituenten tragen, z.B. Halogen wie Fluor, Chlor und Brom, C₁-C₄-Alkyl, vor allem Methyl und Ethyl, C₁-C₄-Alkoxy, vor allem Methoxy und Ethoxy, und Halogenalkyl wie Trifluormethyl sowie auch einen weiteren Chlorformylrest.

Beispiele für bevorzugte Ausgangsverbindungen (III) sind neben 2-Chlorbenzoylchlorid und 3-Methoxybenzoylchlorid vor allem Benzoylchlorid und Isophthalsäurechlorid.

Enthalten die Benzoylhalogenide (III) zwei Halogenformylgruppen, so werden üblicherweise zwei Moleküle Aminoanthrachinon (II) pro Molekül (III) umgesetzt. Umgekehrt kann auch ein zwei oder mehr Aminogruppen enthaltendes Anthrachinon (II) mit zwei oder mehr Molekülen Benzoylhalogenid (III) reagieren.

Die Einsatzmengen an (II) und (III) richten sich üblicherweise nach der Zahl der pro Molekül gewünschten Reaktionen. In der Regel kommen 1 bis 3 mol, bevorzugt 1,3 bis 2 mol, Benzoylhalogenid pro Aminogruppe zum Einsatz.

Das erfindungsgemäße Verfahren wird bevorzugt in Gegenwart eines säurebindenden Mittels durchgeführt. Es ist jedoch auch möglich, das säurebindende Mittel erst nach der Umsetzung bei der Aufarbeitung des Reaktionsgemisches zuzugeben.

Geeignete säurebindende Mittel sind z.B. Erdalkalioxide und -hydroxide wie Magnesiumoxid, Calciumoxid und -hydroxid, Alkalisalze organischer und anorganischer Säuren wie Natriumacetat, Natriumdihydrogen-, Dinatriumhydrogen- und Trinatriumphosphat, Kaliumdihydrogen-, Dikaliumhydrogen- und Trikaliumphosphat, Natrium- und Kaliumhydrogencarbonat und Natrium- und Kaliumcarbonat und Amine, insbesondere tertiäre Amine wie Pyridin, Chinolin und Dimethylanilin sowie auch Mischungen dieser Verbindungen.

Bevorzugt sind hierbei Natriumhydrogencarbonat, Natriumcarbonat, Natriumphosphat und Pyridin.

Üblicherweise werden pro kg Benzoylaminoanthrachinon (I) 0,1 bis 1 kg säurebindendes Mittel eingesetzt.

Die Reaktiongstemperatur hängt von der Reaktivität der Reaktionspartner ab und beträgt im allgemeinen 30 bis 195°C.

Üblicherweise wird man die Umsetzung bei Normaldruck durchführen, man kann jedoch auch unter Druck arbeiten.

Die Reaktionszeiten hängen ebenfalls stark von der Reaktivität der Reaktionspartner ab und liegen in der Regel im Bereich von 1 bis 10 h.

Verfahrenstechnisch geht man beim erfindungsgemäßen Verfahren zweckmäßigerweise so vor, daß man das Aminoanthrachinon (II) im Benzoesäurealkylester suspendiert bzw. löst und das Benzoylhalogenid (III) bei der gewählten Reaktionstemperatur auf einmal oder kontinuierlich, z.B. in 0,5 bis 5 h, zudosiert. Insbesondere bei höheren Reaktionstemperaturen kann es auch von Vorteil sein, (III) bereits vor Erreichen der endgültigen Temperatur zuzugeben. Soll die Umsetzung in Gegenwart eines säurebindenden Mittels vorgenommen werden, so kann dessen Zugabe vor oder nach dem Erhitzen der Mischung aus (II) und dem Benzoesäuremethylester erfolgen.

Die Aufarbeitung des Reaktionsgemisches auf die Produkte gegebenenfalls nach Abkühlen kann auf verschiedene Weise erfolgen. Eine Möglichkeit ist die Wasserdampfdestillation des Gesamtansatzes zur Entfernung und Rückgewinnung des Lösungsmittels durch Phasentrennung und Filtration des Produktes aus der wäßrigen Suspension. Eine weitere Möglichkeit besteht darin, das Lösungsmittel, gegebenenfalls im Vakuum, abzudestillieren, den Rückstand in Wasser anzuschlämmen, das Restlösungsmittel durch Wasserdampfdestillation zu entfernen und zurückzugewinnen sowie das Produkt wieder aus der wäßrigen Suspension abzufiltrieren. Schließlich kann man das Produkt auch direkt, gegebenenfalls nach Zugabe eines Verdünnungsmittels wie Methanol, aus dem Reaktionsgemisch abfiltrieren, dann in Wasser anschlämmen, einer Wasserdampfdestillation zur Entfernung und Rückgewinnung des anhaftenden Lösungsmittels unterziehen und schließlich wieder aus der wäßrigen Suspension abfiltrieren.

Das erfindungsgemäße Verfahren zur Herstellung von Benzoylaminoanthrachinonen zeichnet sich durch eine Reihe von Vorteilen aus. Die Umsetzungen laufen glatt und vollständig ab, weshalb die erhaltenen Produkte von hoher Reinheit sind und direkt zum Färben bzw. zur weiteren Umsetzung zu den Farbstoffen verwendet werden können. Von besonderem Vorteil ist, daß in vielen Fällen eine deutlich konzentriertere Fahrweise als bei Verwendung von Nitrobenzol möglich ist, also deutlich bessere Raum/Zeit-Ausbeuten erzielt werden können.

Das erfindungsgemäße Verfahren stellt also insgesamt eine vorteilhafte, wirtschaftliche, technisch leicht zu verwirklichende und die Umwelt nicht belastende Möglichkeit zur Herstellung von Benzoylaminoanthrachinonen dar.

### Beispiele

### Herstellung verschiedener Benzoylaminoanthrachinone

### Beispiel 1

Ein Gemisch aus 440 g Benzoesäuremethylester, 50 g 1,4-Diaminoanthrachinon und 37 g Natriumhydrogencarbonat wurde unter Rühren auf 40°C erhitzt. Dann wurden 31 g Benzoylchlorid in 6 h zugetropft.

Nach einstündigem Nachrühren bei 40°C wurde das Lösungsmittel durch Wasserdampfdestillation entfernt. Filtration der verbleibenden wäßrigen Suspension, Waschen des Niederschlags mit Wasser und Trocknung ergaben 70 g 1-Amino-4-benzoylaminoanthrachinon (Wertgehalt 88,5 %)

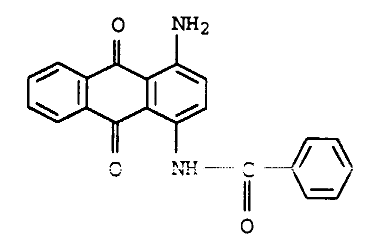

### Beispiel 2

Ein Gemisch aus 540 g Benzoesäuremethylester und 90 g 1,4-Diamino-2,2'-(5',6'-phthaloylbenzthiazolyl)anthrachinon wurde unter Rühren auf 90°C erhitzt. Nach Zugabe von 6,5 g Pyridin und Zutropfen von 72 g Benzoylchlorid in 0,5 h wurde das Reaktionsgemisch in 2 h auf 140°C erhitzt und unter Rühren 3 h bei dieser Temperatur gehalten.

Nach Abkühlen des Reaktionsgemisches auf 60°C, Zugabe von 45 g Natriumhydrogencarbonat und Überführen in einen Schaufeltrockner wurde das Lösungsmittel im Vakuum abdestilliert. Der Rückstand wurde in 800 g Wasser angerührt, 3 h bei 70°C gehalten, abfiltriert, gewaschen und getrocknet.

Es wurden 100 g des Farbstoffs C.I. Vat Blue 31 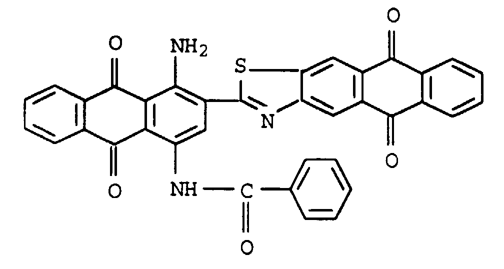 erhalten.

### Beispiel 3

Eine Suspension aus 1400 g Benzoesäuremethylester und 360 g 4,4'-Diamino-1,1'-dianthrimid wurde unter Rühren auf 125°C erhitzt. Nach Zugabe von 280 g Benzoylchlorid in 1 h wurde die Mischung noch 5 h bei 150°C gerührt.

Nach Abkühlen und Neutralisation durch Zugabe von 48 g Natriumcarbonat wurde das Lösungsmittel im Schaufeltrockner abdestilliert. Der Rückstand wurde in 5000 g Wasser 3 h bei 50°C ausgerührt, abfiltriert, gewaschen und getrocknet.

Es wurden 439 g 4,4'-Dibenzoylamino-1,1'-dianthrimid (Wertgehalt 75 %) 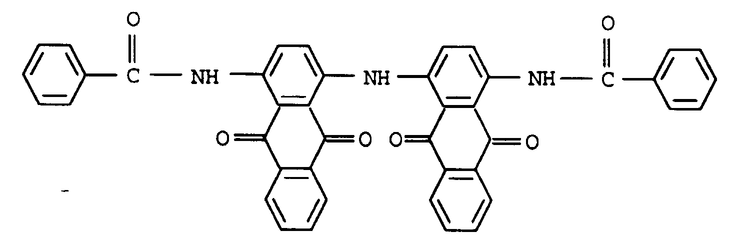 erhalten.

Das Produkt ist Vorstufe für den Küpenfarbstoff C.I. Vat Black 27.

### Beispiel 4

Eine Suspension aus 2350 g Benzoesäuremethylester und 400 g (9)-Benzo-5-aminoanthrachinoyl(2,1-h)-8-azapyrenon-3 wurde unter Rühren auf 55°C erhitzt. Nach Zugabe von 307 g Benzoylchlorid wurde die Reaktionsmischung auf 150-155°C erhitzt und unter Rühren 6 h bei dieser Temperatur gehalten.

Nach Abkühlen und Zugabe von 250 g Natriumhydrogencarbonat wurde das Lösungsmittel mit Wasserdampf abdestilliert. Filtrieren, Waschen und Trocknen ergaben 456 g des Küpenoliv-Farbstoffs

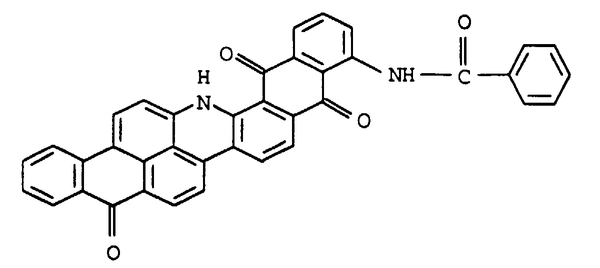

### Beispiel 5

Ein Gemisch aus 600 g Benzoesäuremethylester und 125 g 1-Aminoanthrachinon wurde auf 145°C erhitzt und in 30 min mit einer Lösung von 58 g Isophthalsäurechlorid in 50 g Benzoesäuremethylester versetzt.

Nach einstündigem Nachrühren bei 145°C wurde das Reaktionsgemisch auf 25-30°C abgekühlt und filtriert. Das Filtergut wurde zweimal mit je 50 g Benzoesäuremethylester gewaschen. Nach Entfernen des Lösungsmittels durch Wasserdampfdestillation, Filtrieren, Waschen und Trocknen wurden 148 g C.I. Vat Yellow 26 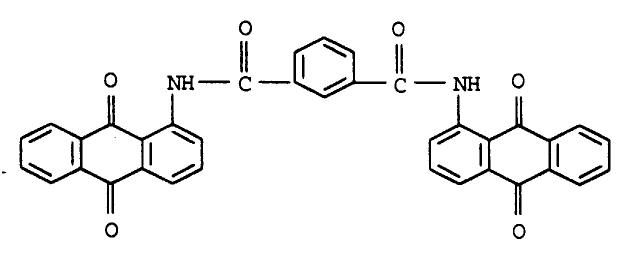 erhalten.

### Beispiel 6

Ein Gemisch aus 350 g Benzoesäuremethylester und 50 g 1,4-Diamino-2,2'-(5',6'-phthaloylbenzthiazolyl)anthrachinon wurde unter Rühren auf 150°C erhitzt. Nach Zugabe von 30 g m-Trifluormethylbenzoylchlorid wurde die Reaktionsmischung in 1 h auf 190-195°C erhitzt und 4 h unter Rühren bei dieser Temperatur gehalten.

Der nach Abkühlen auf 100°C und Filtration isolierte Rückstand wurde mit 50 g Benzoesäuremethylester gewaschen und in Wasser angerührt. Nach Entfernen des Lösungsmittels durch Wasserdampfdestillation, Filtrieren und Trocknen wurden 63 g C.I. Vat Blue 30 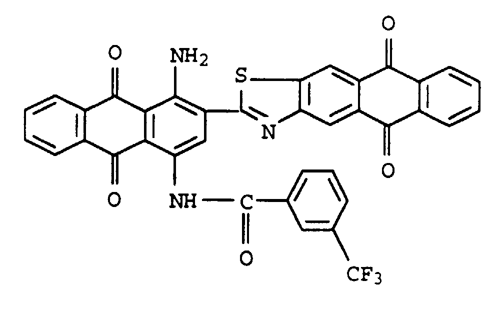 erhalten.

### Beispiel 7

Ein Gemisch aus 250 g Benzoesäuremethylester, 24 g 1,5-Diaminoanthrachinon und 25 g Natriumcarbonat wurde unter Rühren auf 140°C erhitzt. Nach Zutropfen von 19 g Benzoylchlorid wurde die Reaktionsmischung weitere 2 h bei 140°C gerührt.

Nach Abkühlen auf 120°C wurde die Mischung über ein beheiztes Filteraggregat filtriert und der Filterrückstand mit 150 g Benzoesäuremethylester von 120°C gewaschen. Das Filtrat wurde mit 300 g Methanol verdünnt und 10 h bei Raumtemperatur gerührt. Das nach Filtration der Suspension erhaltene Filtergut wurde zunächst mit 50 g Methanol und dann mit 50 g Wasser gewaschen und getrocknet.

Es wurden 17 g 1-Amino-5-benzoylaminoanthrachinon (Wertgehalt 94,6 %) 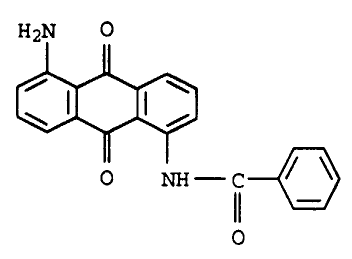 erhalten.

## Patentansprüche

1. Verfahren zur Herstellung von Benzoylaminoanthrachinonverbindungen (I) durch Umsetzung von mindestens eine primäre Aminogruppe enthaltenden Anthrachinonverbindungen (II) mit Benzoylhalogeniden (III) in Gegenwart eines organischen Lösungsmittels, dadurch gekennzeichnet, daß man als organisches Lösungsmittel einen Benzoesäure-C₁-C₆-alkylester verwendet.

2. Verfahren nach Anspruch 1, dadurch gekennzeichnet, daß man als organisches Lösungsmittel Benzoesäuremethylester verwendet.

3. Verfahren nach Anspruch 1 oder 2, dadurch gekennzeichnet, daß man die Umsetzung in Gegenwart eines säurebindenden Mittels durchführt.

## Claims

1. A process for preparing benzoylaminoanthraquinone compounds (I) by reaction of anthraquinone compounds (II) containing at least one primary amino group with benzoyl halides (III) in the presence of an organic solvent, wherein the organic solvent used is a C₁-C₆-alkyl benzoate.

2. A process as claimed in claim 1, wherein the organic solvent used is methyl benzoate.

3. A process as claimed in claim 1 or 2, wherein the reaction is carried out in the presence of an acid acceptor.

## Revendications

1. Procédé de préparation de composés benzoylaminoanthraquinoniques (I) par réaction de composés anthraquinoniques (II) contenant au moins un groupement amine primaire, avec des halogénures de benzoyle (III), en présence d'un solvant organique, caractérisé en ce que l'on utilise un ester alkylique en C₁-C₆ de l'acide benzoïque en tant que solvant organique.

2. Procédé selon la revendication 1, caractérisé en ce que l'on utilise le benzoate de méthyle en tant que solvant organique.

3. Procédé selon la revendication 1 ou 2, caractérisé en ce que l'on effectue la réaction en présence d'un agent piégeant les acides.
